(19) Europäisches Patentamt / European Patent Office / Office européen des brevets

(11) **EP 3 072 470 A1**

(12) **EUROPEAN PATENT APPLICATION**
published in accordance with Art. 153(4) EPC

(43) Date of publication:
**28.09.2016 Bulletin 2016/39**

(51) Int Cl.:
***A61B 18/20*** (2006.01)

(21) Application number: **14863361.3**

(22) Date of filing: **18.11.2014**

(86) International application number:
**PCT/JP2014/080516**

(87) International publication number:
**WO 2015/076262 (28.05.2015 Gazette 2015/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.11.2013 JP 2013239219**

(71) Applicant: **Arai Medphoton Research Laboratories, Corporation**
**Kawasaki-shi, Kanagawa 211-0063 (JP)**

(72) Inventors:
• **ARAI, Tsunenori**
**Kawasaki-shi**
**Kanagawa 211-0063 (JP)**
• **ITO, Arisa**
**Kawasaki-shi**
**Kanagawa 211-0012 (JP)**
• **KIMURA, Takehiro**
**Tokyo 160-8582 (JP)**

(74) Representative: **Grünecker Patent- und Rechtsanwälte**
**PartG mbB**
**Leopoldstraße 4**
**80802 München (DE)**

(54) **MEDICAL TOOL AND PHOTOTHERAPEUTIC DEVICE**

(57)    The present invention provides a medical device and a phototherapy system that include a light diffuser that transmits light to a site to be treated and electrodes for measuring electric potential and that allow phototherapy and measurement of electric potential using a single device.

A medical device 30 is inserted into a living body and transmit light to a site to be treated to treat the site. The medical device includes a distal end portion 44t that has a long tube 41 and a long outer wall of the distal end portion, the wall being provided with electrodes 49T and 49R exposed on the outer surface of the tube 41, and long light-diffusers disposed inside the outer wall of the distal end portion. The distal end portion 44t includes a light transmission equalizer 49h for equalizing light transmission along the length direction of the outer wall of the distal end portion.

FIG. 7

EP 3 072 470 A1

**Description**

TECHNICAL FIELD

The present invention relates to a medical device that includes a light diffuser for diffusing light toward a site to be treated and that is used by inserting the medical device into a living body and a phototherapy system that includes the medical device.

BACKGROUND ART

Electrode catheters are commonly used as a medical device for diagnosing or treating cardiac arrhythmia. Among them, an electrode catheter for measuring electric potential at, for example, the pulmonary vein of a heart, the catheter having a loop distal-end portion, is known (for example, Patent Literature 1).

The electrode catheter of the Patent Literature I includes a catheter body that has at least one lumen, a control handle that is connected to the proximal end of the catheter body, and the distal end portion of the catheter, the portion having a lumen that is connected to the distal end of the catheter body and that communicates with the at least one lumen of the catheter body and being formed into a circular loop shape. The distal end portion of the catheter has a plurality of ring electrodes disposed on the outer circumference of the distal end portion, and a tip electrode disposed at the tip of the distal end portion.

According to the invention of the Patent Literature 1, the catheter includes the distal end portion that is formed into a loop shape and a plurality of ring electrodes that are disposed on the outer circumference of the distal end portion, and thus electric potential can be measured almost over the entire area of the distal end portion of the catheter.

In addition, a hand-held ablation device that is used to form conduction blocks around a pulmonary vein to treat arrhythmias, the ablation device using radiation such as infrared radiation to form photoablative lesions, is known (for example, Patent Literature 2).

The ablation device of the Patent Literature 2 is used in epicardial application performed during a surgical procedure that involves opening a patient's chest cavity or endocardial application performed during a valve replacement procedure that involves opening the chest to expose the heart muscle and the ablation device includes, within the loop distal end portion, a light diffuser that extends along the length direction of the distal end portion.

The ablation device of the Patent Literature 2 can form a linear lesion having a length that corresponds to the length of the light diffuser and can more readily form a line that blocks abnormal electrical conduction.

CITATION LIST

PATENT LITERATURE

PATENT LITERATURE 1: JP 2012-034852 A
PATENT LITERATURE 2: JP 2008-501441 A

SUMMARY OF INVENTION

TECHNICAL PROBLEM

However, the electrode catheter of the Patent Literature 1 and the ablation device of the Patent Literature 2 respectively have only the single function of measuring electric potential and the single function of photoablation. Thus, it has been necessary to use both of the electrode catheter and the ablation device to, for example, form conduction block around a pulmonary vein.

A medical device that includes electrodes for measuring electric potential, the electrodes being formed around a light diffuser for diffusing light toward a site to be treated, has not been known.

The present invention has been made in view of the foregoing problem. An object of the present invention is to provide a medical device and a phototherapy system that include a light diffuser for diffusing light toward a site to be treated and electrodes for measuring electric potential and that allow phototherapy and measurement of electric potential using a single device.

SOLUTION TO PROBLEM

The problem is solved by the medical device of the present invention for treatment of a site to be treated by inserting the medical device into a living body and irradiating the site with light, the medical device including an distal end portion that has a long tube and a long outer wall of the distal end portion, the wall being provided with electrodes exposed on the outer surface of the tube, and long light-diffusers disposed inside the outer wall of the distal end portion. The distal end portion includes a light transmission equalizer that equalizes light transmission along the length direction of the outer wall of the distal end portion.

Such configuration allows prevention of light diffused by the light diffuser from being non-uniformly transmitted due to the electrodes disposed on the outer surface, even though the electrodes are disposed on the outer surface of the tube that includes a long light-diffuser therein. This allows provision of a medical device that includes a light diffuser for irradiation and electrodes for measuring electric potential.

While light irradiation therapy is performed by the light diffuser for irradiation, the same medical device can be used to measure electric potential. As light irradiation and measurement of electric potential can be performed by a single medical device, the surgery operation is simplified, and physical burden on the patient is also reduced.

The electrodes may include a plurality of approximately annular elements that are spaced along the length direction of the distal end portion over the entire distal end portion extending from one end in the length direction to the other.

Such configuration allows prevention of light diffused by the light diffuser from being non-uniformly transmitted due to the electrodes. As the plurality of electrodes are spaced along the length direction of the distal end portion, electric potential can be measured in a balanced manner over the entire length of the medical device that includes the long light-diffuser.

As the electrodes include approximately annular elements, the electrodes can be readily fixed to the medical device.

The electrodes may have a length of 1 mm or less and preferably 0.6 mm or less in the longitudinal direction of the distal end portion.

Such configuration allows an area of the outer circumference of the distal end portion covered by the electrodes to be smaller, which can prevent light diffused by the light diffuser for irradiation from being blocked by the electrodes.

The approximately annular elements may be annular elements and may be provided with a plurality of voids penetrating through the annular elements.

The voids may be holes or slits.

Such configuration allows transmission of light through the voids, which can prevent light diffused by the light diffuser for irradiation from being blocked by the electrodes. This can prevent light diffused by the light diffuser from being non-uniformly transmitted due to the electrodes.

In the tube, a blocking member for blocking light diffused by the light diffuser may also be disposed along the length direction of the tube, and the voids may be disposed only on the side extending from the blocking member to the light diffuser.

In such configuration, the voids are provided in the region in which light diffused by the light diffuser for irradiation needs to be transmitted in order to prevent the light from being blocked, while the voids are not provided in the region in which light diffused by the light diffuser for irradiation does not need to be transmitted. Thus, electric potential can be measured stably.

The voids penetrating through the electrodes can be readily formed, and the balance between the amount of transmission of light diffused by the light diffuser for irradiation and the stability of measurement of electric potential by the electrodes can be designed freely by selecting the size and the position of the holes to be formed.

The distal end portion may include a loop portion that has a coil shape having one and a few convolutions when the portion is subject to no external stresses and a bent portion at an end of the loop portion, and the voids may be formed only in the outer peripheral side of the coil shape.

When light is radially irradiated to form a line that blocks abnormal electrical conduction in treatment of arrhythmias, such configuration allows transmission of sufficient light toward the outer peripheral side of the coil shape of the distal end portion, the outer peripheral side being the direction in which light is to be transmitted.

The approximately annular elements may be formed of a coil having a wire diameter of from 0.05 to 1 mm.

The coil may have one or more convolutions or a pitch that is larger than the wire diameter.

Such configuration allows more stable prevention of light diffused by the light diffuser from being blocked by the electrodes.

If the pitch is larger than the wire diameter, light can be transmitted through the gaps between the convolutions of the coil of the electrodes, and thus light diffused by the light diffuser for irradiation can be prevented from being blocked by the electrodes.

The tube may include spiral projections formed along the outer circumference of the tube, and the projections may be spaced in a plural number on the outer surface of the tube along the length direction of the distal end portion over the entire distal end portion extending from one end in the length direction to the other. And the electrodes may be fixed to the projections.

Such configuration allows the electrode to be readily contacted with surrounding tissue such as a tissue to be treated and allows the contact area to be increased when the electrodes are contacted with the tissue. Thus, this can reduce blockage of light due to the electrodes formed of a narrow coil and can prevent decrease in accuracy in detecting electric potential due to the electrodes formed of a narrow coil.

The electrodes may be a light-transmissive metal film that is continuously disposed along the direction of extension of the tube and that has a film thickness of from 1 to 300 nm.

Such configuration allows light diffused by the light diffuser to be transmitted through the light-transmissive metal film and allows light transmission to be equalized over the entire length of the distal end portion.

The photodynamic therapy system may include the medical device, a light source that provides excitation light to the light diffuser for irradiation of the medical device, a detection unit that detects the electric potential of the electrodes, and a calculator that receives a signal indicative of the detected electric potential from the detection unit and that calculates the electric potential of the electrodes based on the signal.

EFFECTS OF INVENTION

According to the present invention, the electrodes disposed on the outer surface can prevent light diffused by the light diffuser from being non-uniformly transmitted, even though the electrodes are disposed on the outer surface of the tube that includes a long light-diffuser therein. This allows provision of a medical device that includes a light diffuser for irradiation and electrodes for measuring electric potential.

Thus, while light irradiation therapy is performed by the light diffuser for irradiation, the same medical device can be used to measure electric potential. As light irradiation and measurement of electric potential can be performed by using a single medical device, the surgery operation is simplified, and physical burden on the patient is also reduced.

BRIEF DESCRIPTION OF DRAWINGS

FIG. 1 is a block diagram illustrating a schematic configuration of a laser catheter and a treatment apparatus according to an embodiment of the present invention.

FIG. 2 is a graph illustrating a concept of PBI (photosensitizer bleaching index).

FIG. 3 is a graph illustrating the change in fluorescence detector output over time in extracellular photodynamic therapy using a laser catheter according to an embodiment of the present invention.

FIG. 4 is a schematic illustration of use of a laser catheter according to an embodiment of the present invention in treatment of arrhythmias.

FIG. 5 is an illustration of a head portion of a laser catheter according to an embodiment of the present invention.

FIG. 6 is a cross-sectional view taken along the line B-B in FIG. 5.

FIG. 7 is a vertical cross-sectional view of a head portion of a laser catheter according to an embodiment of the present invention.

FIG. 8 is a schematic illustration of a light diffuser for irradiation according to an embodiment of the present invention.

FIG. 9 is a cross-sectional view of a laser catheter of FIG. 6 with an electrode fixed to the outer circumference of the laser catheter.

FIG. 10 is an illustration of a head portion of a laser catheter according to a modified embodiment of the present invention.

FIG. 11 is a vertical cross-sectional view of a laser catheter according to a modified embodiment of the present invention.

FIG. 12 is an illustration of a head portion of a laser catheter according to another modified embodiment of the present invention.

FIG 13 is a vertical cross-sectional view of a laser catheter according to another modified embodiment of the present invention.

FIG. 14 is an enlarged partial view illustrating fixation of a coil electrode to a projection in FIG. 13.

FIG. 15 is a cross-sectional view illustrating a modified embodiment of fixation of a coil electrode.

FIG. 16 is a cross-sectional view illustrating another modified embodiment of fixation of a coil electrode.

FIG. 17 is an illustration of a head portion of a laser catheter according to still another modified embodiment of the present invention.

FIG. 18 is a vertical cross-sectional view of a laser catheter according to still another modified embodiment of the present invention.
FIG. 19 is a cross-sectional view of a head portion of a laser catheter according to still another modified embodiment of the present invention.

DESCRIPTION OF EMBODIMENTS

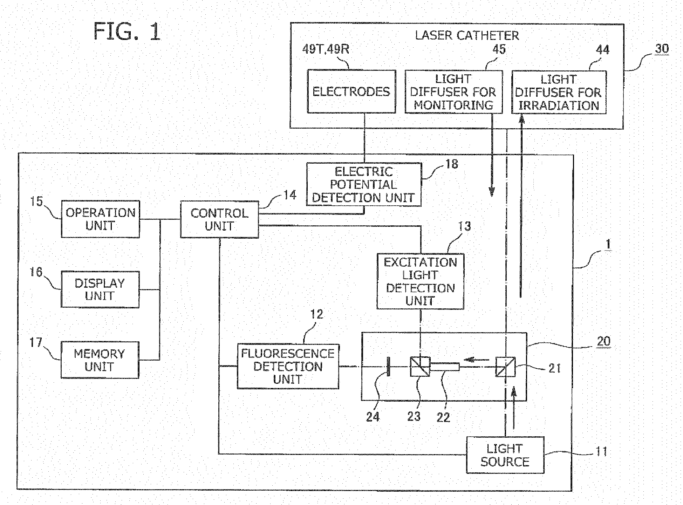

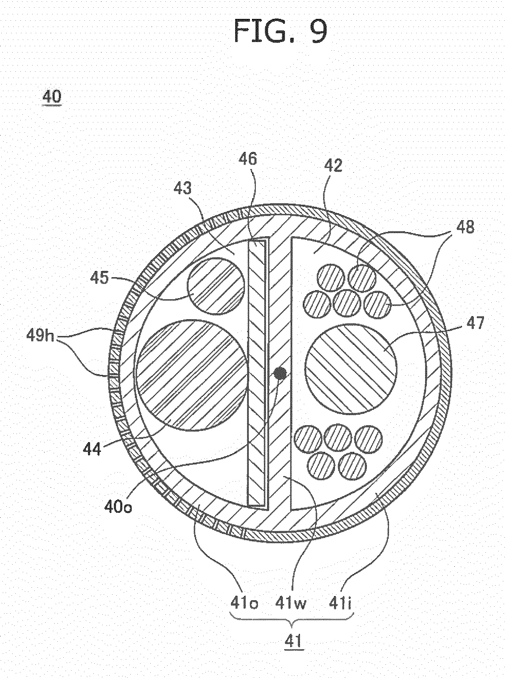

Now, a laser catheter 30, which is a medical device according to an embodiment of the present invention, will be described with reference to FIGS. 1-19.

Although the laser catheter 30 will be described as the medical device in the embodiment, the medical device is not limited thereto and may be any medical device that is introduced into an in vivo tissue to be treated, such as a sheath, an endoscope, a venous conduit, an arterial conduit, a bronchoscope, a cystoscope, a culpascope, a colonoscope, a trocar, a laparoscope, or another medical tube.

Although embodiments herein describe an exemplary use of the medical device of the present invention to treat arrhythmias by creating a line that blocks abnormal electrical conduction using a photodynamic therapy, use of the medical device is not limited thereto, and the medical device can be used in various therapies such as, for example, a photodynamic therapy for pancreatic cancer or cholangiocarcinoma using an endoscope having a very small diameter such as a choledochoscope (having an outer peripheral side diameter of from 1 to 3 mm) or a pancreatoscope (having an outer peripheral side diameter of from 1 to 2.5 mm).

The medical device of the present invention can be used for any clinical condition that can be treated using a medical device such as the laser catheter 30 that includes a light emitting probe. For example, the medical device of the present invention can be used in a photodynamic therapy for cancers, infections, or arteriosclerosis, or antithrombotic therapy using a laser catheter. The medical device of the present invention can also be used in any therapies using laser radiation or laser measurement.

As used herein, the proximal side of, for example, the laser catheter 30 refers to a side that is closer to the outside of a living body, i.e., the clinician when the laser catheter 30 is in the living body. The distal side of the laser catheter 30 refers to a side that is closer to the catheter's end inserted in a living body, i.e., that is closer to a tissue to be treated.

As illustrated in FIG. 1, the laser catheter 30 of this embodiment is used in combination with a treatment apparatus 1. A combination of the laser catheter 30 and the treatment apparatus 1 corresponds to the phototherapy system of emboidments.

The treatment apparatus 1 is an apparatus for an extracellular photodynamic therapy (extracellular PDT), which is carried out by distributing a sufficient amount of a photosensitizing agent (hereinafter referred to as PDT agent) in extracellular interstitial spaces and blood vessels in a tissue to be treated as the site to be treated of embodiments. The treatment apparatus 1 transmits light for a photodynamic therapy through a light diffuser for irradiation of the laser catheter 30 to carry out a photodynamic therapy and also receives the returned fluorescence from a light diffuser for monitoring and calculates and displays an index of information on the progress of the therapy in real time during the therapy. Based on the index of information on the progress of the therapy displayed on a display in real time during the therapy, the clinician can estimate, at any time, the extent of lesions by light irradiation in a tissue to be treated.

The extracellular photodynamic therapy in this embodiment is carried out by distributing a PDT agent in extracellular components, i.e., extracellular fluid and/or cell-permeant fluid in a living body and continuously delivering the PDT agent by vascular permeation.

Although the treatment apparatus 1 is used for treatment of arrhythmias in this embodiment, the treatment apparatus 1 may be used in other therapies such as photodynamic therapies for infections, as long as the therapies are an extracellular photodynamic therapy carried out by distributing a sufficient amount of a PDT agent in extracellular interstitial spaces and blood vessels in a tissue to be treated. The treatment apparatus 1 can also be used for treatment of cancers (including gastrointestinal cancers, respiratory cancers, brain cancers, and skin cancers), treatment of arteriosclerosis, and the like by accumulating a PDT agent in cells in a tissue to be treated, as long as a sufficient amount of the PDT agent and oxygen are continuously delivered to extracellular interstitial spaces and blood vessels in the tissue to be treated. The treatment apparatus 1 may also be used in angioplasty.

Arrhythmias treated by using the treatment apparatus 1 of this embodiment include arrhythmias caused by the presence of an abnormal electrical conduction site or abnormal excitation site including, in particular, any tachyarrhythmias conventionally treated by radiofrequency ablation. In particular, the treatment apparatus 1 can be used for paroxysmal supraventricular tachycardia including atrial fibrillation (AF) including paroxysmal atrial fibrillation (paroxysmal AF), persistent atrial fibrillation (persistent AF), and permanent atrial fibrillation (permanent AF), atrial flutter (AFL), atrioventricular reciprocating tachycardia (AVRT), atrioventricular nodal reentrant tachycardia (AVNRT), and atrial tachycardia (AT).

The infections include MRSA infections, gingivitis, periodontitis, peri-implantitis, herpes, stomatitis, candidosis, and the like.

The PDT agent used in this embodiment is a photosensitizing agent.

Unlike photodynamic therapies for cancers, accumulation of the agent in the tissue is not necessary in this embodiment, and thus the drug-light interval between drug administration and light irradiation is short, ranging from several minutes to several tens of minutes, and the treatment is started within a short period of time after drug administration. Thus, it is preferred to use a photosensitizing agent that is distributed in the interstitial spaces rapidly after administration such as by intravenous injection, that is quickly excreted, and that is water-soluble.

In particular, examples of the photosensitizing agent include ATX-S10, which is a chlorin-based agent having a chlorin moiety (670 nm) (an iminochlorinaspartic acid derivative, Oriental Menthol Industry Ltd., The right was assigned to Photochemical Co., Ltd. in 2000, Japanese Unexamined Patent Application Publication No. H06-80671), NPe6 (664 nm) (talaporfin sodium, Laserphyrin®, mono-L-aspartyl chlorin e6, Japanese Patent No. 2961074), mTHPC (652 nm), SnET2 (660 nm) (tin etiopurpurin, Miravant Medical Technologies, Inc.), AlPcS (675 nm) (chloro aluminum sulphonated phthalocyanine), BPD-MA (690 nm) (benzoporphyrin derivative monoacid ring A, QLT Inc.), and Lu-tex (732 nm) (lutetium texaphyrin). Among them, talaporfin sodium is preferred.

«Principle and Reaction Mechanism of Extracellular Photodynamic Therapy for Arrhythmias»

In the photodynamic therapy for arrhythmias in this embodiment, a photosensitization reaction is used for treatment within a short period of time after administration of a PDT agent, i.e., at a point where the PDT agent is distributed more in an extracellular compartment (interstitial spaces) than in an intracellular compartment.

First, a PDT agent is administered by, for example, intravenous injection, and then the PDT agent is distributed in the extracellular interstitial spaces and blood vessels at a high concentration. In the extracellular compartment at this point, a sufficient amount of the PDT agent and oxygen are consistently and continuously delivered by the blood flow. The PDT agent is delivered by permeation through the blood vessel wall into the interstitial spaces.

After a short drug-light interval between drug administration and light irradiation, ranging from several minutes to several tens of minutes, a target site is irradiated with laser light using transcatheter techniques to provide sufficient light by the laser beams while a sufficient amount of the PDT agent and oxygen are delivered by the blood flow, thereby causing a photosensitization reaction among the PDT agent, the light, and the oxygen.

In the photosensitization reaction, the PDT agent is excited with light irradiation. The energy of the excited PDT agent is transferred to the oxygen in the extracellular compartment, and reactive singlet oxygen (reactive oxygen) is produced.

The total production of the singlet oxygen is the integral of the irradiation time and the amount of singlet oxygen per unit time.

Production of the excited singlet oxygen leads to destruction of a protein bound by the PDT agent, conversion of the excited singlet oxygen to triplet ground state oxygen, and bleaching of the agent due to high oxidizing potential of the singlet oxygen, which provides the therapeutic effects of blocking the electrical conduction of the cardiomyocytes and causing necrosis due to oxidative damage to ion channels and cell membranes.

As used herein, the term bleaching of the agent refers to destruction of a photosensitizing agent as a PDT agent by the singlet oxygen. The bleaching of the agent is proportional to a change in fluorescence of the PDT agent per unit time.

«PBI (Photosensitizer Bleaching Index)»

In this embodiment, PBI (photosensitizer bleaching index) is used as an index of treatment depth in the extracellular photodynamic therapy.

In the extracellular photosensitivity reaction occurring in the extracellular photodynamic therapy, the blood flow delivers a sufficient amount of the PDT agent during the therapy, and thus the integral of a photosensitizer bleaching value and time is considered to be the total amount of bleached photosensitizer at that point in time, i.e., the total production of singlet oxygen as a therapeutically active component.

In this embodiment, PBI determined by the following Formula 1 is used as an index that corresponds to the total bleaching amount.

$$PBI = (fluo(0) - fluo(t_d))t_d \qquad \text{(Formula 1)}$$

In the formula, the symbols have the following meanings:

fluo(t): intensity of returned fluorescence at time t

6

$t_d$: light irradiation time [s]

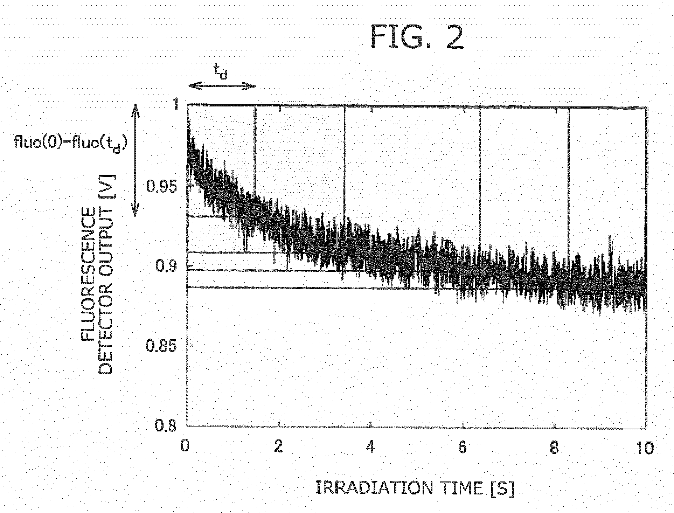

The concept of PBI will be described with reference to FIG. 2. FIG. 2 is a graph illustrating the change in the intensity of the returned fluorescence over time in the extracellular photosensitivity reaction superimposed on a plurality of rectangles that conceptually indicate PBI,

PBI is the product of a difference between the fluorescence intensity at a light irradiation time $t_d$, which indicates a measurement time, and the fluorescence intensity at the time of start of light irradiation $t_0$ and the light irradiation time $t_d$, which is a period from the start time to the measurement time.

In the FIG. 2, the length of the sides of the plurality of rectangles along the abscissa corresponds to the light irradiation time $t_d$, which is a period from the start time to a measurement time.

At the time of start of light irradiation to, i.e., at the time 0 in the FIG. 2, light irradiation is not started. Thus, the photosensitizer is still not bleached, and the intensity of the returned fluorescence fluo(t) is the highest. After the time 0, light irradiation is started and the photosensitizer is more bleached and exhibits a lower fluo(t) at a later measurement time $t_d$.

Thus, the length of the sides of each of the rectangles along the ordinate corresponds to the difference between the fluorescence intensity at the light irradiation time $t_d$, which indicates a measurement time, and the fluorescence intensity at the time of start of light irradiation to.

Then, PBI corresponds to the area of each of the rectangles in the FIG. 2.

In the Formula 1, it is assumed that a sufficient amount of oxygen and the PDT agent are delivered at a constant rate by permeation through the blood vessel wall into the interstitial spaces. Thus, the amount of the supplied light energy is a rate-controlling factor.

A measurement of fluorescence derives from both of the PDT agent in blood and the PDT agent in the interstitial spaces, and it is assumed that the ratio of the agent in blood and the agent in the interstitial spaces is constant.

It is assumed that a production of singlet oxygen is proportional to an amount of the supplied light energy in the extracellular photosensitivity reaction. Thus, the estimated necrosis depth, i.e., estimated treatment depth $d_{nec}$, by the extracellular photosensitivity reaction depends on light distribution in the depth direction.

Accordingly, the estimated treatment depth $d_{nec}$ can be represented as a logarithm of PBI as determined by the following Formula 2 obtained by modifying the general formula $E_{(d)} = I_0 \cdot \exp(-\mu_{eff}d)$, which is used for determining an absorption coefficient.

$$d_{nec} = \frac{1}{\mu_{eff}} \ln\left(\frac{k(t)I_0 PBI}{E}\right)$$

$$= \frac{1}{\mu_{eff}} \left\{ \ln(PBI) + \ln\left(\frac{k(t)I_0}{E}\right) \right\}$$

$$\text{(Formula 2)}$$

In the formula, the symbols have the following meanings:

$d_{ncc}$: Lesion depth [mm]
$\mu_{eff}$: Attenuation coefficient [/mm]
$k(t)$: Constant changing over time: $1/\{fluo(0) - fluo(t)\}$
$I_0$: Intensity of light at tissue surface [mW]
$E$: Energy of light at depth [J]

«Correction of PBI»

The PBI determined by the Formula 1 can be converted to a more practical and more useful index by computing a corrected PBI.

Methods for correcting PBI are classified into methods for correcting PBI by using the waveform itself of the change in fluorescence intensity over time and methods for correcting PBI by using histophysiological information or the like obtained by another process.

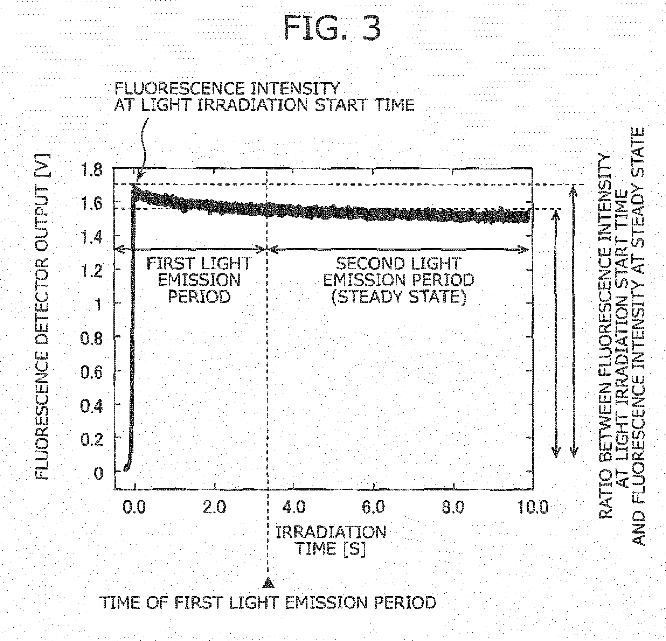

The methods for correcting PBI by using the waveform of the change in fluorescence intensity over time will be described with reference to the following methods 1, 2, and 3 and a graph of the FIG. 3. FIG. 3 is a graph illustrating the change in fluorescence detector output over time in extracellular photodynamic therapy.

As illustrated in FIG 3, it has been confirmed that in the extracellular photodynamic therapy, the fluorescence intensity detected by a fluorescence detection unit 12 in FIG. 1 reaches a steady state some time after light irradiation is started.

Thus, the period from start of light irradiation to termination of light irradiation can be separated into a first light emission period from start of light irradiation to achievement of steady-state fluorescence intensity and a second light emission period from the end of the first light emission period to termination of light irradiation in the steady state.

○ Correction Method 1

A corrected PBI can be obtained by multiplying a PBI determined by the Formula 1 by the fluorescence intensity output at the light irradiation start time.

The fluorescence intensity at the light irradiation start time reflects the concentration of the PDT agent, and thus in this method, the PBI can be corrected for concentration of the PDT agent distributed in the tissue.

○ Correction Method 2

The ratio between the fluorescence intensity at the light irradiation start time and the fluorescence intensity in the steady state indicates that in the first light emission period, the intensity is in a transient state before reaching the steady state.

The PBI indicates a reaction extent in the steady state, and thus the PBI is corrected by using two indexes, which are the first light emission period and the ratio between the fluorescence intensity at the light irradiation start time and the fluorescence intensity in the steady state, to reflect the photosensitization reaction in a transient state in the first light emission period, depending on the conditions.

In the first light emission period, the balance of delivery of the PDT agent and oxygen by vascular permeation or the blood flow and consumption of the PDT agent and oxygen by the photodynamic therapy is different from the balance in the second light emission period, which is in the steady state.

Thus, the Correction Method 2 can provide a corrected PBI that is consistent with reality where the balance of delivery of the PDT agent and oxygen by vascular permeation or the blood flow and consumption of the PDT agent and oxygen by the photodynamic therapy in the first light emission period, which is in a transient state, is different from the balance in the steady state.

○ Correction Method 3

A corrected PBI can be obtained by dividing a PBI determined by the Formula 1 by the intensity of returned excitation light at an irradiation time.

In this method, PBI can be corrected for difference in optical properties in each tissue to be treated.

In the methods for correcting PBI by using histophysiological information or the like obtained by another process, PBI is corrected by using histophysiological information obtained by measurement using another process, information on the general condition of the patient obtained using a common device for monitoring a living body, or a known physiological value for a tissue to be treated.

The physiological information, the information on the general condition, the physiological value are, for example, oxygen levels such as $PaO_2$ (arterial oxygen tension) and $SpO_2$ (peripheral capillary oxygen saturation), amounts of hemoglobins such as the total amount of hemoglobin, the amount of oxygenated hemoglobin, and the amount of deoxygenated hemoglobin, blood flow rate, blood flow volume, vessel density per unit volume, and blood flow volume in each tissue, body temperature of the patient, temperature of a tissue to be treated, and levels of serum proteins such as albumin, HDL (high-density lipoprotein), and LDL (low-density lipoprotein).

Among them, the oxygen levels are a factor that has an effect on reaction efficiency and oxygen delivery. The amounts of hemoglobins are a factor that has an effect on oxygen delivery. The blood flow volume and the blood flow rate are a factor that has an effect on delivery of a PDT agent and oxygen. The temperature is a factor that has an effect on reaction efficiency. The levels of serum proteins have an effect on the delivery amount of a PDT agent, as serum proteins bind to PDT agents.

«Configuration of Treatment Apparatus 1»

Now, the treatment apparatus 1 of this embodiment will be described with reference to FIG. 1.

FIG. 1 is a block diagram illustrating a schematic configuration of the treatment apparatus 1 of this embodiment.

The treatment apparatus 1 includes a light source 11, an optical system 20, a fluorescence detection unit 12, a returned excitation excitation light detection unit 13, a control unit 14, a operation unit 15, a display unit 16, a memory unit 17, and an electric potential detection unit 18.

The light source 11 emits light for exciting a PDT agent. The light emitted by the light source 11 has a wavelength equal to an absorption wavelength in the Q band of the PDT agent. In this embodiment, talaporfin sodium is used as the PDT agent, and thus a device that emits light at a wavelength in the range of from 400 to 700 nm and preferably of 660 nm is used as the light source 11. It is to be understood that if an agent other than talaporfin sodium is used as the PDT agent, the wavelength of light emitted by the light source 11 is not limited to the above range.

As the light source 11, a laser device such as a semiconductor laser device, a light-emitting diode light source, or a lamp light source is used.

The excitation light emitted by the light source 11 enters a light diffuser for irradiation 44 of the laser catheter 30 through the optical system 20. The light source 11 preferably has an input power of 1000 mW or less and preferably 500 mW or less and preferably has an output of 150 mW/cm or less and preferably 75 mW/cm or less over the length of the light diffuser for irradiation 44 of the laser catheter 30. Such configuration allows light diffused by the light diffuser for irradiation 44 to be transmitted at an appropriate intensity, which can prevent excessive penetration.

The optical system 20 injects the excitation light emitted by the light source 11 through a polarizing beam splitter 21 into the light diffuser for irradiation 44 of the laser catheter 30. The optical system 20 extracts fluorescence emitted by a PDT agent irradiated with the excitation light and the excitation light returned from the laser catheter 30 from a light diffuser for monitoring 45, which is a light diffuser for receiving light in the laser catheter 30, and respectively injects the fluorescence and the returned light into the fluorescence detection unit 12 and the returned excitation light detection unit 13.

Although the light diffuser for monitoring 45 only receives light in this embodiment, the light diffuser for monitoring 45 is not limited thereto and may diffuse the excitation light for measurement purposes.

The optical system 20 includes the polarizing beam splitter 21, an optical fiber 22, a dichroic mirror 23, and a band-pass filter 24.

The excitation light emitted by the light source 11 enters the light diffuser for irradiation 44 of the laser catheter 30. Part of the excitation light emitted by the light source 11 is reflected by side edge surfaces of an optical fiber 44f and a connector (not shown) that connects the treatment apparatus 1 to the laser catheter 30 and the distal end portion of the laser catheter 30 and enters the polarizing beam splitter 21 as the returned excitation light.

The fluorescence diffused by the light diffuser for monitoring 45 in the laser catheter 30 also enters the polarizing beam splitter 21.

The polarizing beam splitter 21 reflects light incident from the light diffuser for monitoring 45 in the laser catheter 30 and directs the light through the optical fiber 22 to the dichroic mirror 23.

The dichroic mirror 23 reflects the light having a wavelength equal to or less than a predetermined wavelength in the range of from 680 to 690 nm and transmits the light having a wavelength equal to or more than a predetermined wavelength in the range of from 680 to 690 nm. Thus, the dichroic mirror 23 transmits fluorescence having a peak wavelength of about 710 nm and directs the fluorescence through the band-pass filter 24 to the fluorescence detection unit 12, while the dichroic mirror 23 reflects the returned excitation light having a peak wavelength of about 663 nm and directs the light to the returned excitation light detection unit 13.

The band-pass filter 24 is a filter that transmits light having a wavelength of $710 \pm 2$ nm.

Although talaporfin sodium is used as the PDT agent, and thus the dichroic mirror 23 and the band-pass filter 24 are configured to transmit and reflect light having a wavelength as specified above in this embodiment, the dichroic mirror 23 and the band-pass filter 24 are not limited thereto if an agent other than talaporfin sodium is used as the PDT agent.

The fluorescence detection unit 12 and the returned excitation light detection unit 13 include a known linear image detection unit and respectively detect the fluorescence emitted by the PDT agent and injected by the optical system 20 and the returned excitation light extracted from the laser catheter 30. The fluorescence detection unit 12 and the returned excitation light detection unit 13 respectively transmit the detected fluorescence and the returned excitation light as an electrical signal to the control unit 14.

As described above, the control unit obtains information not only on the fluorescence, but also on the returned excitation light, and thus the control unit can be used as a monitor for the operation of the treatment apparatus 1 to determine whether the excitation light used for the therapy is appropriately transmitted under predetermined conditions such as light intensity.

The fluorescence detection unit 12 includes a detection unit element such as a silicon photodiode, an avalanche photodiode, a spectrograph, or a photomultiplier tube. The detection unit element operates at at a sampling rate of from 10 to 1000 Hz and preferably from 100 to 500 Hz.

The control unit 14 controls each of the components in the treatment apparatus 1.

The control unit 14 calculates the intensity of the fluorescence and the intensity of the returned excitation light based on electrical signals received from the fluorescence detection unit 12 and the returned excitation light detection unit 13 and calculates information on the therapy and the tissue in real time based on these intensities.

The control unit 14 calculates information on treatment of a treated tissue in respective portions in a direction of extension of a loop portion 40c of the laser catheter 30 by a known process in real time based on an electric potential signal received from the electric potential detection unit 18.

The control unit 14 also outputs a display instruction that causes the display unit 16 to present results of the computations.

The memory unit 17 is a non-volatile memory and is configured as, for example, a flash memory, a HDD, or another solid-state memory. The control unit 14 associates the calculated intensity of the fluorescence, the calculated intensity of the excitation light, information on the extent of lesions, and time information received from a timer (not shown) for measuring the amount of time elapsed since a reference time such as the time of start of radiation of excitation light with each other and records the associated data as the change over time in the memory unit 17.

The display unit 16 is a display device that uses, for example, a liquid crystal display. After receiving a display instruction from the control unit 14, the display unit 16 displays information of, for example, the extent of lesions and time on a display screen in real time, based on display information included in the display instruction.

The operation unit 15 receives an instruction input by the clinician and outputs the received instruction to the control unit 14. The instruction is, for example, an instruction on on/off of the excitation light emitted by the light source 11 or switching of the intensity.

The electric potential detection unit 18 applies a predetermined voltage to electrodes 49 disposed on the outer circumference of the laser catheter 30 and detects information on electric potential of the electrodes 49.

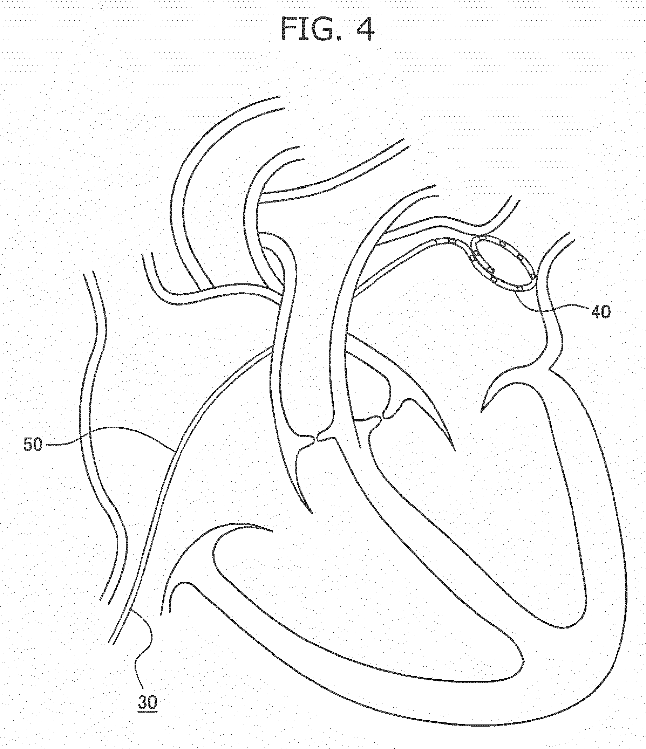

The treatment apparatus 1 is removably connected to the laser catheter 30 illustrated in FIGS. 4-9 via a connector (not shown) connected to an end of a tube (not shown) connected to the treatment apparatus 1.

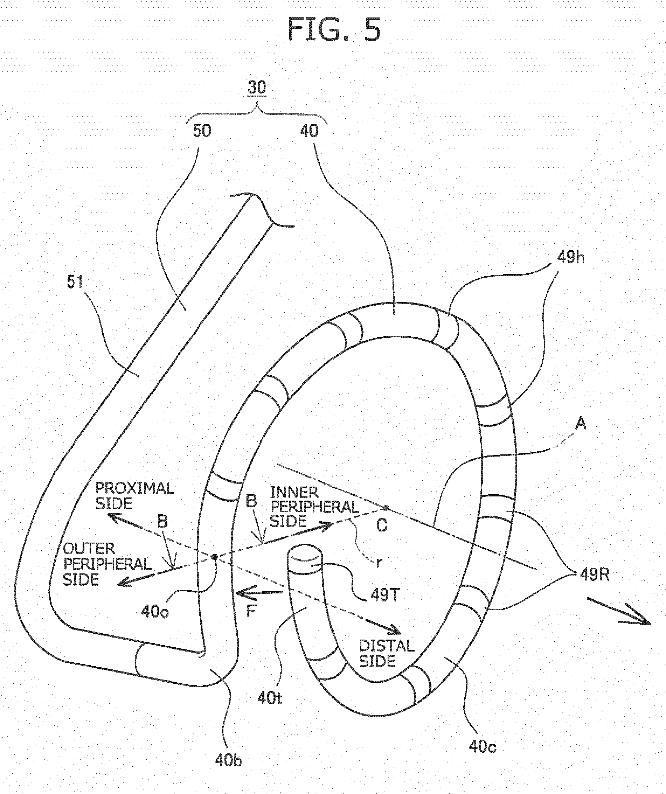

As illustrate in FIG. 4 and FIG. 5, the laser catheter 30 includes a head portion 40 disposed at the distal end of the laser catheter 30 and a long tubular portion 50 connected to the head portion 40 and deposed in proximity to the head portion 40. A control handle (not shown) is connected to a proximal portion of the tubular portion 50 opposite to the head portion 40. A connector (not shown) is disposed in a proximal portion of the control handle opposite to the tubular portion 50.

«Configuration of Head portion 40»

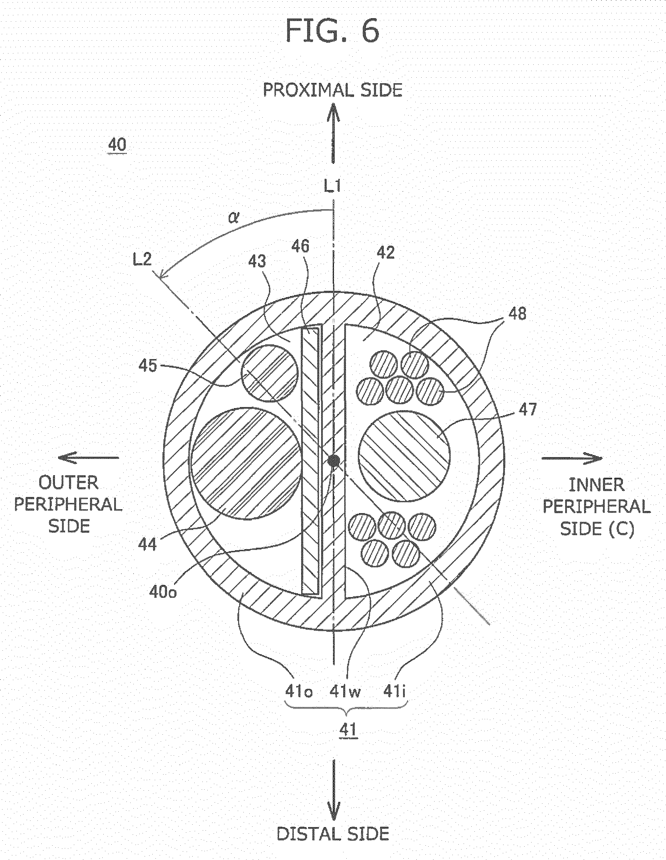

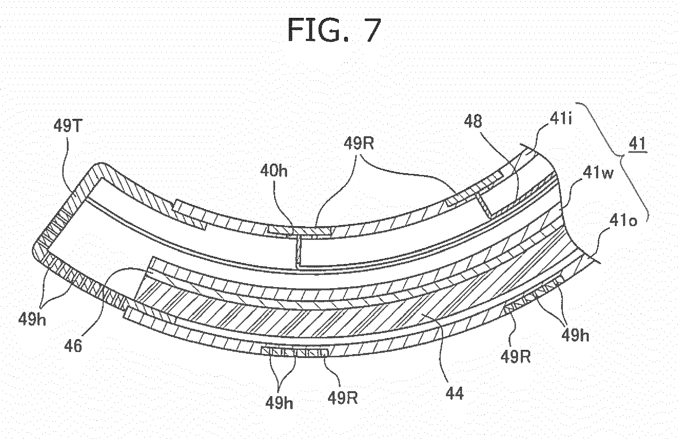

As illustrated in FIG. 6 and FIG. 7, the head portion 40 includes the light diffuser for irradiation 44, the light diffuser for monitoring 45, a reflector 46, a shape-memory wire 47, and lead wires 48 inserted in a transparent tube 41 as a body of a medical device, the tube being shaped of a hollow flexible transparent material. The shape-memory wire 47 allows a loop shape having one and a few convolutions. The shape of the head portion 40 of the laser catheter 30 is also called a lasso shape. Although the shape of the head portion 40 is not a closed circle in a strict sense, the shape is a quasi circular shape, and thus is called a ring shape or a circular shape.

The transparent tube 41 is a soft, hollow, cylindrical, and transparent tube that is formed of a polyether block amide copolymer (PEBAX® from Arkema, Inc.) or the like. Alternatively, the transparent tube 41 may also be formed of an optically-transparent and non-electroconductive polymeric materials such as polyethylene terephthalate (PET), polytetrafluoroethylene (PTFE), fluorinated ethylene propylene (FEP), perfluoroalkoxy (PFA), urethane, polyurethane, or polyvinyl chloride (PVC).

As illustrated in FIG 6 and FIG. 7, the transparent tube 41 includes an inner peripheral side lumen 42 and an outer peripheral side lumen 43 separated by a partition wall 41w.

Although the entire of the transparent tube 41 is formed of a transparent tube in this embodiment, the tube 41 it not limited thereto as long as at least an outer peripheral side wall 41o around the outer peripheral side lumen 43 is transparent. Thus, an inner peripheral side wall 41i and the partition wall 41w may be formed of a semi-transparent or opaque material.

The inner peripheral side lumen 42 is a lumen that is disposed on the inner peripheral side, which faces the center C of the coil shape, and that has an approximately semicircular cross section and is defined by the coil-shaped inner peripheral side wall 41i of the transparent tube 41 and the partition wall 41w. In the inner peripheral side lumen 42, the shape-memory wire 47 is inserted at the longitudinal center of the cross section so that the wire 47 faces to the wall 41i and the partition wall 41w, and ten lead wires 48 are inserted on longitudinally opposite sides of the cross section so that the shape-memory wire 47 is sandwiched between the lead wires 48.

The shape-memory wire 47 is a wire that is formed of a nickel titanium alloy having an approximately circular cross section and that has shape-memory property. The shape-memory wire 47 may be formed of an iron-based shape-memory alloy such as iron-manganese-silicon alloy or a bimetal, which is formed of two metal sheets joined together, each metal having a different coefficient of thermal expansion.

The shape-memory wire 47 has a coil shape having about 1.1 convolutions, the shape having a curved end that is bent at an angle of 90° or more so that the head portion 40 holds a shape as illustrated in FIG. 4 and FIG. 5 when the head portion is subject to no external stresses.

Although the shape-memory wire 47 is omitted in FIG. 7 to facilitate understanding of the drawing, the distal end of the shape-memory wire 47 is fixed to an end electrode 49T, while the proximal end is fixed to the tubular portion 50.

The shape-memory wire 47 is elastic, and when the wire is subject to externally applied pressure, the wire can deform in response to the pressure. Thus, when the head portion 40 is in a blood vessel, pressure from the side wall of the blood vessel causes the wire to take a shape such as a gentle curve conforming to the shape of the blood vessel.

The lead wires 48 are formed of a lead wire used in common electrode catheters, and each of the ten lead wires 48 is connected, by a known method, to one of nine ring electrodes 49R or an end electrode 49T formed on the outer circumference of the head portion 40.

The outer peripheral side lumen 43 is a lumen that is disposed on the outer peripheral side, which faces the side opposite to the center C of the coil shape, and that has an approximately semicircular cross section and is defined by the wall 41o on the outer peripheral side of the coil shape of the transparent tube 41 and the partition wall 41w. In the outer peripheral side lumen 43, the reflector 46 is disposed in contact with the partition wall 41w. The light diffuser for irradiation 44 is inserted at the longitudinal center of the cross section so that the light diffuser faces to the wall 41o and the reflector 46, and the light diffuser for monitoring 45 is inserted adjacent to the light diffuser for irradiation 44.

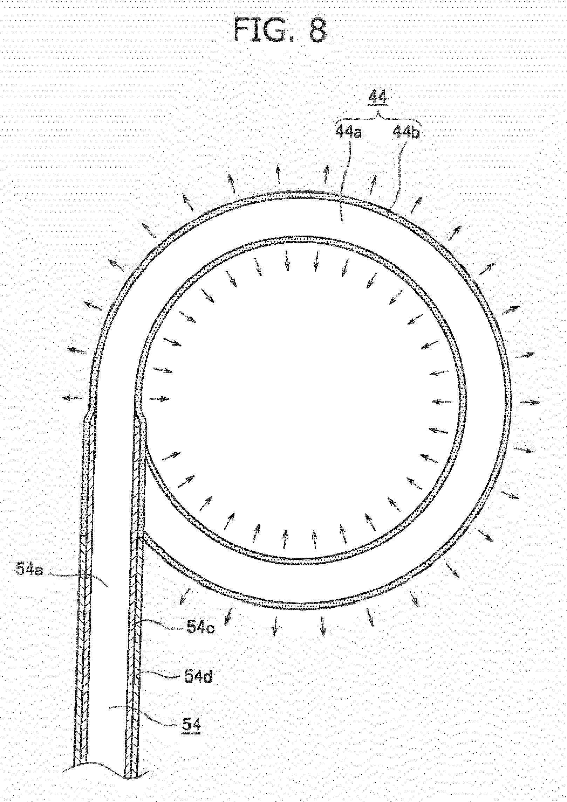

As illustrated in FIG. 8, the light diffuser for irradiation 44 includes a light diffuser body 44a that is formed by removing a cladding 54c and a coating 54d from an optical fiber cable 54 inserted in the tubular portion 50 to expose a core over a predetermined length from an distal end portion 44t and a resin layer 44b that covers the outer circumference of the light diffuser body 44a.

The light diffuser body 44a is integrally formed with the core 54a of the optical fiber cable 54 inserted in the tubular portion 50 and is sandblasted so as to uniformly diffuse light laterally at an angle relative to the direction of the length of the light diffuser body 44a. The light diffuser body 44a is not limited thereto and may include a hollow portion disposed in the center of the body and a light reflecting mirror disposed on the inner surface of the hollow portion or notches in the inner surface of the hollow portion so as to uniformly diffuse light laterally.

The resin layer 44b is formed by, for example, applying ultraviolet-curable acrylic resin having quartz micropowder dispersed therein and curing the resin by ultraviolet light.

A connector (not shown) is fixed to an end of the optical fiber cable 54 opposite to the light diffuser for irradiation 44. The connector allows the fiber cable to be connected to a laser generator (not shown) that includes a laser source (not shown).

The light diffuser for irradiation 44 is not limited to the type in FIG. 8, and another type of light diffusers can also be used.

Methods for forming the light diffuser for irradiation 44 are broadly classified into methods of extending the core 54a of the optical fiber cable 54 to form the light diffuser for irradiation 44 and methods of forming the light diffuser for irradiation 44 separately from the core 54a. Either method may be used to form the light diffuser for irradiation 44 of this embodiment.

In the former methods, the core 54a may be or may not be of a diffusing material itself. In particular, the methods are broadly classified into transmitted-light leakage methods (such as a method of creating small scratches in the cladding 54c to expose part of the core 54a and a method of generating leakage by bending) and methods of using a diffusing material.

The transmitted light leakage methods include abrasive treatment (such as sandblasting, stamping, and solvent treatment), fiber bragg grating (FBG), microbending, and the like.

The methods of using a diffusing material include a method of incorporating a diffusing material in the core 54a/the cladding 54c, a method of exposing the core 54a and introducing a diffusing material in the coating 54d, and the like. The sandblasting is the process of propelling of fine particles and thus is also encompassed in the methods of using a diffusing material.

An example of the latter methods, in which the light diffuser for irradiation 44 is formed separately from the core 54a, is a case in which an optical element that is different than the core 54a is used as the light diffuser for irradiation 44. For example, an optical element such as a polyhedral prism, SELFOC® lens (gradient index lens) is used as the light diffuser for irradiation 44.

The light diffuser for irradiation 44 extends over the entire length of the head portion 40. When the medical device is used for a photodynamic therapy for arrhythmias, the proper diameter of the coil shape in FIG. 5 is in the range of from 5 to 50 mm and preferably from 5 to 15 mm, and the total length of the light diffuser for irradiation 44 is in the range of from 1.5 to 15 cm and preferably from 3 to 9 cm, although the ranges vary depending on the shape of a tissue to be treated.

The light diffuser for irradiation 44 has a diameter of from 0.1 to 1.0 mm and preferably from 0.25 to 0.75 mm.

The light diffuser for irradiation 44 is used to diffuse light emitted by the light source to a site to be treated.

The light diffuser for monitoring 45 has a diameter of from 0.1 to 1.0 mm and preferably from 0.25 to 0.75 mm.

The light diffuser for monitoring 45 has a configuration similar to that of the light diffuser for irradiation 44.

The light diffuser for monitoring 45 is used to receive the returned fluorescence and determine information on the extent of lesions.

The light diffuser for irradiation 44 and the light diffuser for monitoring 45 are adjacent to each other, and thus contact or friction between the diffusers can cause signal degradation due to interference (crosstalk) of signals leaked from the respective diffusers.

Thus, the light diffuser for irradiation 44 and the light diffuser for monitoring 45 may be bonded to each other with a transparent adhesive, or a transparent spacer may be disposed between the diffusers 44 and 45.

Although the light diffuser for irradiation 44 and the light diffuser for monitoring 45 are separately formed in this embodiment, the diffusers may be integrally formed. In particular, the light diffuser for irradiation 44 and the light diffuser for monitoring 45 may be formed of a single light diffuser. In this case, the single light diffuser may be used to diffuse and receive light by switching the diffuser between a diffusing mode and a light-receiving mode based on time.

The reflector 46 is used to prevent light diffused by the light diffuser for monitoring 45 to be directed toward the inner peripheral side of the coil-shaped portion and to cause the light to be directed toward the outer peripheral side. Prevention of the light to be directed toward the inner peripheral side of the coil-shaped portion allows focusing of the light on a tissue to be treated and prevention of irradiation of sites other than the tissue to be treated with the light. This can decrease irradiation of blood present closer to the inner peripheral side and can reduce damage to blood such as hemolysis.

The reflector 46 is formed of a long rectangular aluminum plate having a thickness of from 0.01 to 0.10 mm and more preferably from 0.03 to 0.08 mm and a length that is similar to the length of the light diffuser for irradiation 44 and the light diffuser for monitoring 45. The reflector 46 may be formed of a material such as gold or silver that exhibits a high reflectance for the excitation light. In place of the reflector 46, a curved plate having a U-shaped or V-shaped cross section may be used with the transverse center facing the center C of the coil-shaped portion, or surfaces of the light diffuser for irradiation 44 and the light diffuser for monitoring 45 facing the inner peripheral side of the coil-shaped portion may be plated with a material such as aluminum, gold, or silver.

On the outer circumference of the head portion 40, as illustrated in FIG. 5, nine ring electrodes 49R and an end electrode 49T are spaced at a predetermined interval in the direction of extension of the head portion 40.

The ring electrodes 49R and the end electrode 49T are formed of a biocompatible and electrically conductive material such as platinum, gold, or iridium, or an alloy thereof, or stainless steel. The ring electrodes 49R have a width in the direction of extension of the head portion 40 of 1 mm or less and preferably 0.6 mm or less so as not to block transmission of light.

As illustrated in FIG. 5, FIG. 7, and FIG. 9, the ring electrodes 49R and the end electrode 49T include numerous fine holes 49h in an approximately semicylindrical portion located outer peripheral side to the partition wall 41w, the holes penetrating the ring electrodes 49R and the end electrode 49T in the thickness direction.

The fine holes 49h have, for example, a circular or hexagonal cross section. The ratio of the total opening area of the fine holes 49h, which is determined by adding the opening areas of all of the holes 49h, to the surface area of the approximately semicylindrical portion located outer peripheral side to the partition wall 41w is in the range of from 20 to 95% and preferably from 80 to 95%.

Because the fine holes 49h are provided so that the opening area ratio is in the range of from 20 to 95% and preferably from 80 to 95% as described above, the variation in intensity of light transmitted toward a portion outer peripheral side to the partition wall 41 w in the loop portion 40c is within $\pm 20\%$ in the direction of extension of the transparent tube 41. The fine holes 49h correspond to the light transmission equalizer. In this embodiment, the plurality of ring electrodes 49R and the end electrode 49T are disposed on the outer side of the light diffuser for irradiation 44, and light diffused by the light diffuser for irradiation 44 is partially blocked by the ring electrodes 49R and the end electrode 49. Then, the light transmission tends to differ between the portions with the ring electrodes 49R or the end electrode 49 and the portions without the ring electrodes 49R or the end electrode 49. Thus, the fine holes 49h are provided to prevent the light diffused by the light diffuser for irradiation 44 from being non-uniformly transmitted toward a tissue to be treated.

To prevent absorption of light by the ring electrodes 49R, it is preferred to plate the inner surface of the ring electrodes 49R with a material, such as gold, that has a high reflectance for the irradiated light. It is preferred to form the ring electrodes 49R of a material, such as gold, that has a high reflectance for the irradiated light, also because absorption of light by the ring electrodes 49R is prevented. The layer of a high reflectance material plated on the inner surface of the ring electrodes 49R also corresponds to the light transmission equalizer.

A light-blocking film of a non-conductive material may also be formed between the ring electrode 49R and the end electrode 49T that are adjacent to each other. If the ring electrodes 49R and the end electrode 49T have high light-blocking ability, such configuration allows adjustment of the transmission of the regions having the light-blocking film formed thereon to the transmission of the regions in which the light is blocked by the ring electrodes 49R and the end electrode 49T, which can equalize the transmission in the regions where light is blocked by the ring electrodes 49R or the end electrode 49T and the regions without the ring electrodes 49R and the end electrode 49T. The light-blocking film (not shown) of a non-conductive material also corresponds to the light transmission equalizer.

As illustrated in FIG. 7, side holes 40h are formed in the regions in the head portion 40 where the ring electrodes 49R are to be disposed, and nine lead wires 48 are connected through the side holes 40h to the respective ring electrodes 49R. An end hole is also formed in the distal end of the head portion 40, and a lead wire 48 is connected through the end hole to the end electrode 49T.

The lead wires 48 are formed by coating a metal core wire having a diameter of about 0.075 mm with a resin such as a polyamide-imide resin having a film thickness of about 10 $\mu$m.

Although the head portion includes nine ring electrodes 49R and an end electrode 49T in this embodiment, the number of the ring electrodes 49R is not limited thereto and may be in the range of about from 7 to 9. If the loop portion 40c has a small diameter of about 5 mm, the head portion may include about 4 ring electrodes 49R.

The lead wires 48 are connected via a connector (not shown) to the electric potential detection unit 18 of the treatment apparatus I in FIG. 1. Signals indicative of the electric potential of the ring electrodes 49R and the end electrode 49T are input to the electric potential detection unit 18. Then, the control unit 14 calculates information on treatment of a treated tissue at the respective ring electrodes 49R and the end electrode 49T by a known process, and the display unit 16 displays the information.

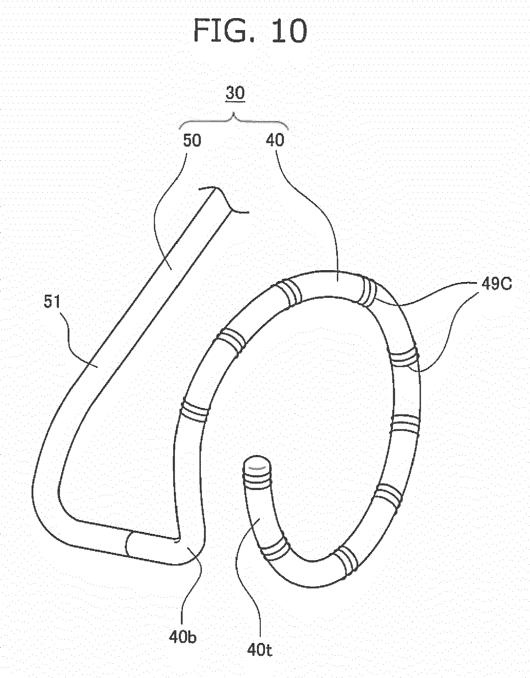

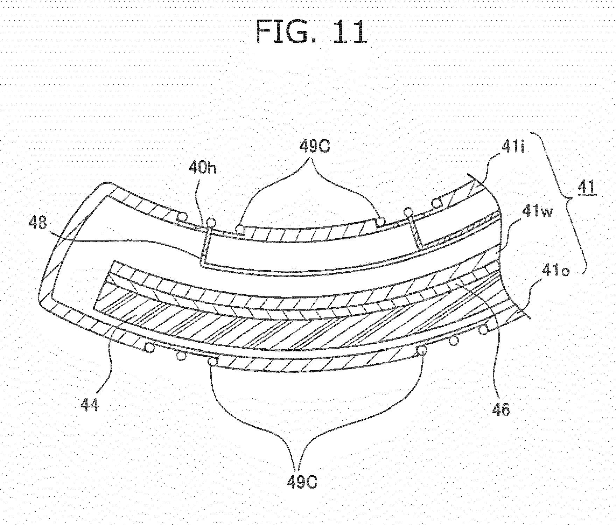

Although the head portion includes the ring electrodes 49R and the end electrode 49T illustrated in FIG. 5, FIG. 7, and FIG. 9 in this embodiment, the head portion may include coil electrodes 49C illustrated in FIG. 10 and FIG. 11 in place of the ring electrodes 49R and the end electrode 49T, as illustrated in the figures.

The coil electrodes 49C in FIG. 10 and FIG. 11 are formed of a coil having a wire diameter of from 0.05 to 1.0 mm and preferably from 0.1 to 0.3 mm and a pitch that is larger than the wire diameter. As used herein, the term pitch refers to the distance from one convolution of the coil to an adjacent convolution. The coil electrodes 49C are formed of a biocompatible and electrically conductive material such as platinum, gold, or iridium, or an alloy thereof, or stainless steel.

Such configuration allows transmission of light through the gaps between the convolutions, and thus highly transmissive electrodes can be formed, while electric potential can be measured due to the coil electrodes 49C.

Although the coil electrodes 49C and the lead wires 48 can be connected to each other by swaging, the coil electrodes 49C are not limited thereto, and the coil electrodes 49C may be formed by pulling the end portion of the lead wires 48 a long distance out of the side hole 40h, peeling the coating on the pulled portion, winding the portion around the transparent tube 41, and fixing the portion to the outer surface of the transparent tube 41 with an adhesive. In this case, the coil electrodes 49C and the lead wires 48 are integrally formed.

Although the coil electrodes 49C in FIG. 10 and FIG. 11 are formed of a wire having a circular cross section, the coil electrodes 49C may be formed of a wire having a rectangular cross section. It is, however, preferred that the coil electrodes 49C have no edges projecting outwardly so as not to inhibit smooth advancement of the laser catheter 30 to a tissue to be treated.

Although the coil electrodes 49C in FIG. 10 and FIG. 11 have 3 convolutions, the coil electrodes are not limited thereto and may have any number of convolutions such as, for example, about 1 to 20 convolutions.

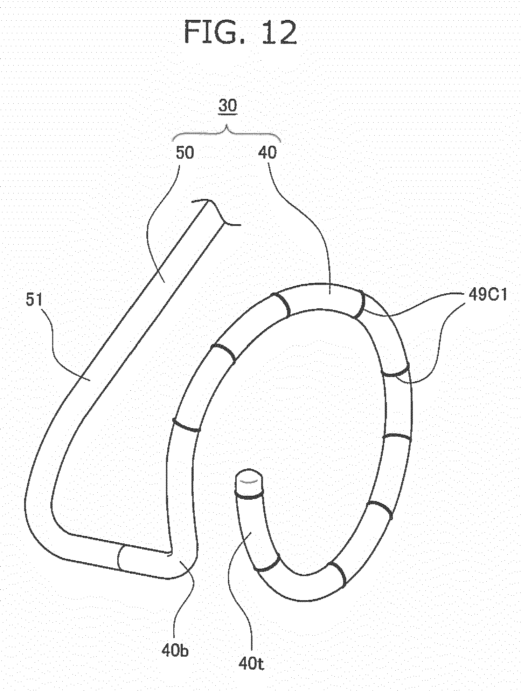

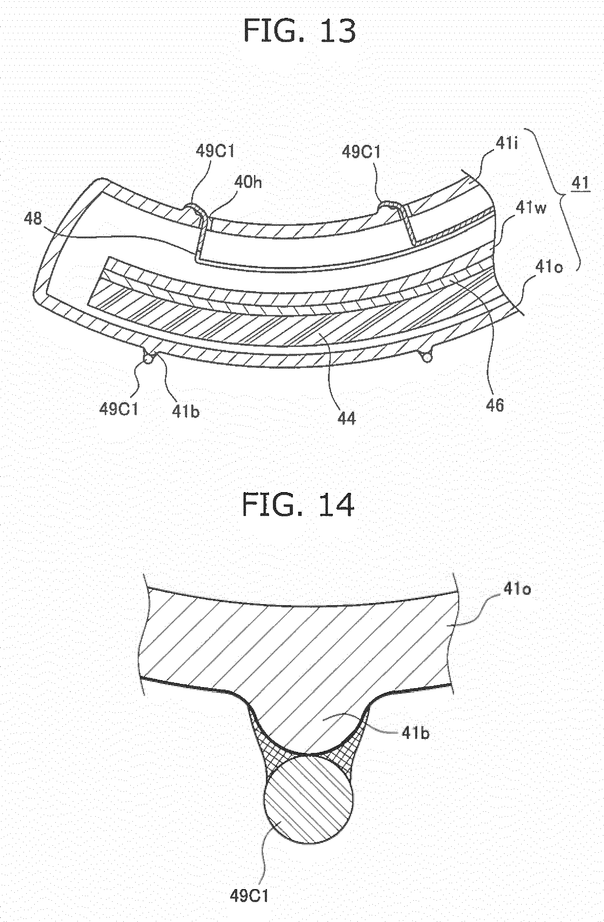

FIG. 12 and FIG. 13 illustrate coil electrodes 49C1 having a single convolution.

The coil electrodes 49C1 are formed by pulling the end portion of the lead wires 48 a long distance out of the side hole 40h, peeling the coating on the pulled portion, winding the portion around the transparent tube 41, and fixing the portion to the outer surface of the transparent tube 41 with an adhesive. Similar to the coil electrodes 49C in FIG. 10 and FIG. 11, the coil electrodes 49C1 may be formed of a coil of a biocompatible and electrically conductive material such as platinum, gold, or iridium, or an alloy thereof, or stainless steel and may be connected to the lead wires 48 by swaging.

The coil electrodes 49C1 are formed of a narrow coil having a single convolution, and thus the area in which electric potential is measured is limited. Then, as illustrated in FIG. 13 and FIG. 14, tubular projections 41b are disposed on the portions of the outer circumference of the transparent tube 41 having the coil electrodes 49C1 wound thereon. The coil electrodes 49C1 may be fixed to the center of the projections 41b with an adhesive. FIG. 14 is an enlarged partial view illustrating fixation of the coil electrodes 49C1 in FIG. 13 to the projections 41b.

As described above, the coil electrodes 49C1 are fixed to the projections 41b, and thus the coil electrodes 49C1 can be readily contacted with an adjacent tissue such as a tissue to be treated, and the contact area can also be increased when the electrodes are contacted with the tissue. This can reduce blockage of light by the coil electrodes 49C1 due to formation of the coil electrodes 49C1 of a narrow coil and can prevent decrease in accuracy in detecting electric potential due to formation of the coil electrodes 49C1 of a narrow coil.

If the coil electrodes 49C1 project from a surface of the transparent tube 41 by a large amount, it may be difficult to advance the medical device due to contact with a surrounding tissue during delivery of the medical device to a site to be treated, depending on the route to a tissue to be treated.

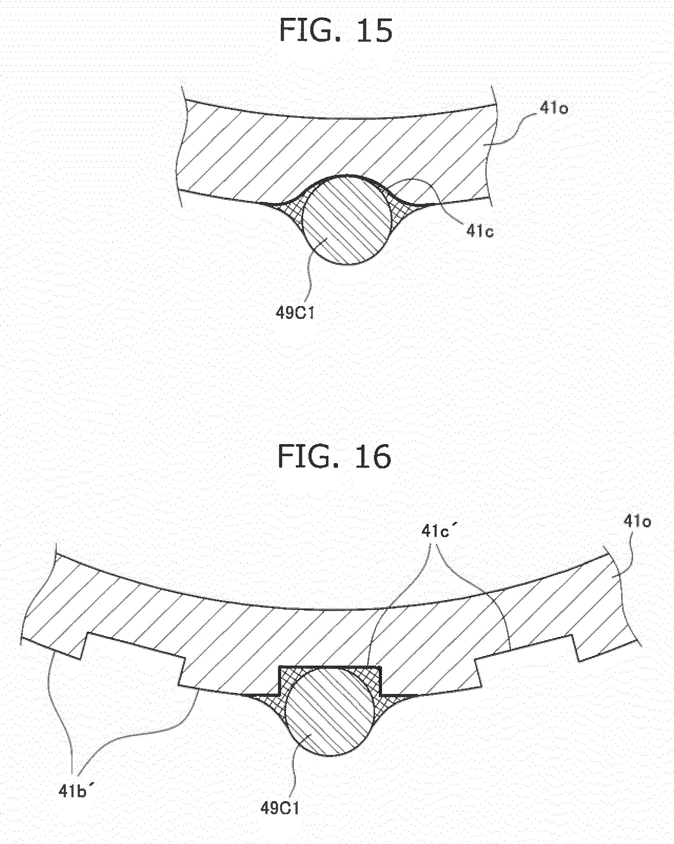

Thus, as illustrated in FIG. 15, it is preferred to dispose recesses 41c having a curved cross section in the portions of the outer circumference of the transparent tube 41 having the coil electrodes 49C1 wound thereon over the entire outer circumference of the transparent tube 41 and to fix the coil electrodes 49C1 with an adhesive so that the coil electrodes 49C1 are partially disposed in the recesses 41c. In this case, the recesses 41c preferably has a depth that is equal to the depth at which the coil electrodes 49C1 are partially disposed in the thickness direction of the electrodes in the recesses with part of the electrodes being exposed, such as, for example, a depth that is between one quarter and one half of the diameter of the coil electrodes 49C1.

As illustrated in FIG 16, annular recesses 41c' extending in a direction perpendicular to the length direction of the transparent tube 41 and protrusions 41 b' may be formed alternately on a surface of the transparent tube 41 along the length direction of the transparent tube 41.

As illustrated in FIG. 16, it is preferred to fix each of the coil electrodes 49C1 to an appropriate recess 41c' of the plurality of recesses 41c' formed alternately with the protrusions 41b' with an adhesive so that the coil electrode 49C1 is partially disposed in the recess 41c. The recesses 41c' preferably have a depth that is equal to the depth at which the coil electrodes 49C1 are partially disposed in the recesses with part of the electrodes being exposed, such as, for example, a depth that is between one quarter and one half of the diameter of the coil electrodes 49C1.

As the coil electrodes 49C and 49C1 have a configuration as illustrated in FIGS. 10-16, the variation in intensity of light transmitted outer peripheral sidely to the partition wall 41w in the loop portion 40c is within $\pm 20\%$ in the direction of extension of the transparent tube 41. The coil electrodes 49C and 49C1 correspond to the light transmission equalizer.

A light-blocking film of a non-conductive material may be formed between the adjacent coil electrodes 49C or between the adjacent coil electrodes 49C1. If the coil electrodes 49C or 49C1 have high light-blocking ability, such configuration allows adjustment of the transmission of the regions having the light-blocking film formed thereon to the transmission of the regions in which the light is blocked by the coil electrodes 49C or 49C1, which can equalize the transmissions in the regions in which light is blocked by the coil electrodes 49C or 49C1 and the regions without the coil electrodes 49C or 49C1. The light-blocking film (not shown) of a non-conductive material also corresponds to the light transmission equalizer.

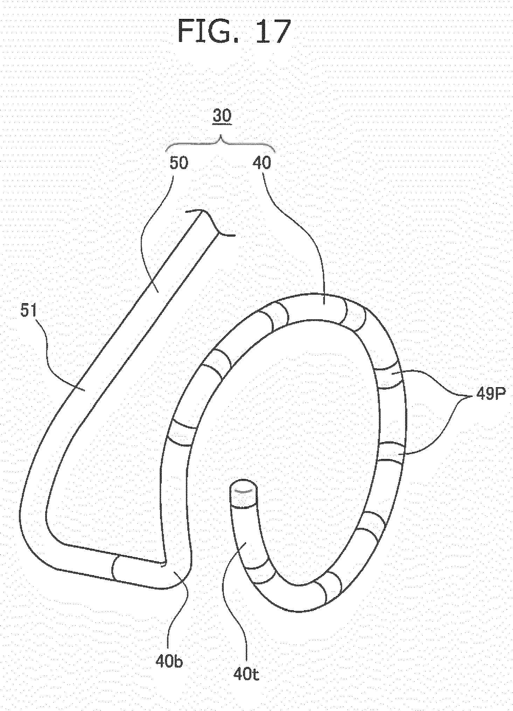

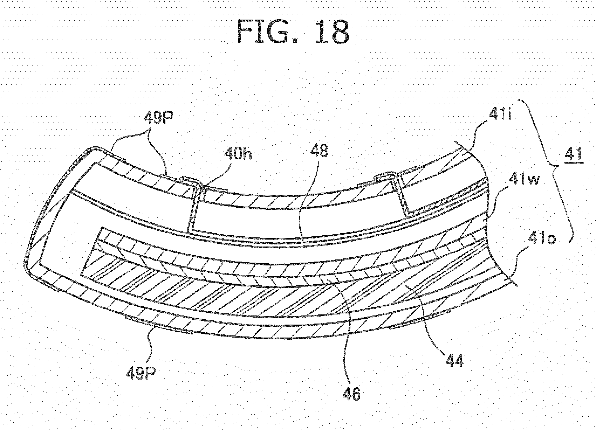

As illustrated in FIG 17 and FIG. 18, the head portion may include plating films 49P of a biocompatible and electrically conductive material such as platinum, gold, or iridium, or an alloy thereof, or stainless steel having a thickness of 1 to 300 nm and preferably from 3 to 150 nm on the outer surface of the transparent tube 41 in the loop portion 40c, in place of the ring electrodes 49R and the end electrode 49T illustrated in FIG. 5, FIG. 7, and FIG. 9.

The plating films 49P are disposed at 10 locations that are spaced apart at a predetermined interval in the direction of extension of the head portion 40. One of the films is disposed at the distal end of the head portion 40 so as to close the opening of the head portion 40, while the remaining nine films are formed as a ring perpendicular to the direction of extension of the head portion 40. The ring-shaped plating films 49P have a width of about 2 mm in the direction of extension of the head portion 40.

A light-blocking film of a non-conductive material may be formed between the adjacent plating films 49P. If the plating films 49P have high light-blocking ability, such configuration allows adjustment of the transmission of the regions having the light-blocking film formed thereon to the transmission of the regions in which the light is blocked by the plating films 49P, which can equalize the transmissions in the regions in which light is blocked by the plating films 49P and the regions without the plating films 49P. The light-blocking film (not shown) of a non-conductive material also corresponds to the light transmission equalizer.

As the plating films 49P have a configuration as illustrated in FIG 17 and FIG. 18, the variation in intensity of light transmitted outer peripheral sidely to the partition wall 41 w in the loop portion 40c is within $\pm 20\%$ in the direction of extension of the transparent tube 41. The plating film 49P corresponds to the light transmission equalizer.

Because the head portion 40 includes the shape-memory wire 47, the distal end portion forms the loop portion 40c that has a coil shape having about 1.1 convolutions, and the head portion 40 has a bent portion 40b at the proximal end of the loop portion 40c, the bentj portion being bent at an angle of from about 90° to 105°, as illustrated in FIG. 5, when the head portion is subject to no external stresses.

Application of a force F that lightly pushes the distal end portion 40t of the loop portion 40c toward the bent portion 40b along the direction of the central axis A of the loop portion 40c in the head portion 40 causes the distal end portion 40t to contact the bent portion 40b and causes the head portion 40 to form a closed circle.

Thus, simply by lightly pushing the head portion 40 against a tubular site to be treated such as a vein as illustrated in FIG. 4, the head portion 40 can be caused to form a closed circle, and the internal light diffuser for irradiation 44 can also be caused to form a closed circle, which can form a gap-free line that blocks abnormal electrical conduction.

The head portion 40 may be configured so that the head portion 40 forms a circle with the distal end portion 40t and the bent portion 40b being in contact with each other when the head portion is subject to no external stresses.

In the example in FIG. 6, the partition wall 41w and the reflector 46 are formed along a straight line L1 parallel to the central axis A of the loop portion 40c in FIG. 5. Thus, the straight line L1 is perpendicular to a straight line r extending from the center 40o of a traverse cross section of the head portion 40 to the center C the loop portion 40c. However, the partition wall 41 w and the reflector 46 do not need to be formed along the straight line L1, and the partition wall 41 w and the reflector 46 may be formed along a straight line L2 in FIG. 6 that is inclined at an angle $\alpha$ ($0° \leq \alpha \leq 90°$) with respect to the straight line L1.

FIG. 6 illustrates a case in which α is 0°. In this case, the partition wall 41 w and the reflector 46 separate the transparent tube 41 into an inner peripheral side and an outer peripheral side along a plane parallel to the central axis A. Thus, light diffused by the light diffuser for irradiation 44 is transmitted toward the outer peripheral side in a direction approximately perpendicular to the straight line L1. Thus, the case of FIG. 6 in which α is 0°, is suitable, for example, when the entire part of the head portion 40 is inserted into a blood vessel that is slightly thicker than the head portion 40, and then light is transmitted from the head portion 40 toward the outer peripheral side.

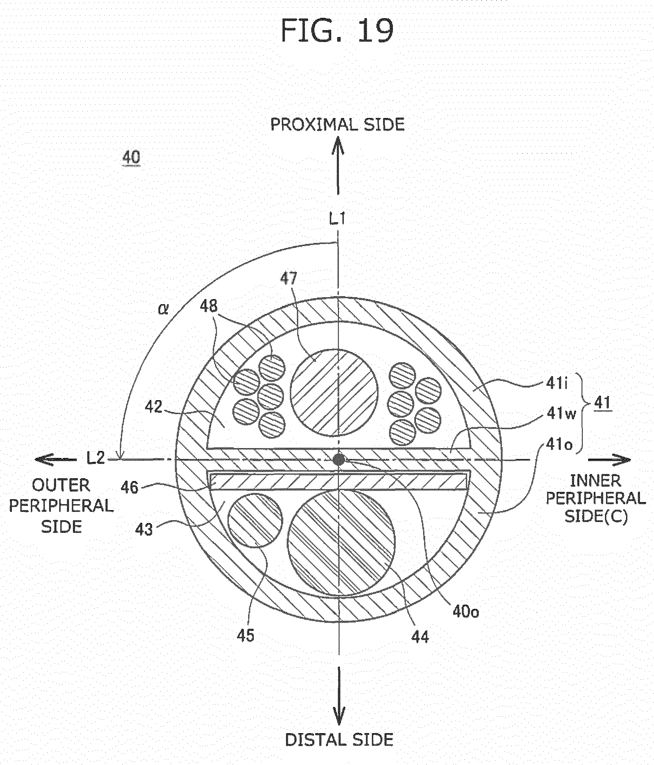

FIG. 19 illustrates a case in which α is 90°. In this case, the partition wall 41 w and the reflector 46 separate the transparent tube 41 into a proximal side and a distal side along a plane perpendicular to the central axis A. Thus, light diffused by the light diffuser for irradiation 44 is transmitted toward the distal side in a direction along the straight line L1. Thus, the case of FIG. 19 in which α is 90° is suitable, for example, when the distal side of the head portion 50 having a small coil shape having a diameter of about 5 mm is pushed against a site to be treated to irradiate a narrow punctate region.

The partition wall 41 w and the reflector 46 may be formed along the L2 that is inclined at an angle α (0° ≤ α ≤ 90°) with respect to the L1, similar to the L2 in FIG. 6. In this case, the partition wall 41 w and the reflector 46 separate the transparent tube 41 into a proximal and inner peripheral side and a distal and outer peripheral side along a plane that is inclined at an angle of from more than 0° to less than 90° with respect to the central axis A. Unlike the case in which the partition wall 41w and the reflector 46 are formed along the L2 in FIG. 6, the partition wall 41w is inclined with respect to the straight line L1 so that an end of the partition wall 41 w facing a tissue to be treated (the distal end) is disposed on the inner peripheral side of the coil shape. For example, when the loop portion 40c is pushed from the left atrial side toward the inner surface of the pulmonary vein, the tissue to be treated is positioned not on the exact outer peripheral side of the loop portion 40c but slightly distal to the outer peripheral side, and thus inclination of the partition wall 41w and the reflector 46 toward the distal and outer peripheral side allows more efficient light irradiation and measurement.

The tubular portion 50 includes the optical fiber cable 54 continuous with the light diffuser for irradiation 44, an optical fiber cable (not shown) continuous with the light diffuser for monitoring 45, and ten lead wires 48 inserted in a tube 51, which acts as an enclosure of the tubular portion 50.

The tube 51 is formed of a resin such as polyether block amide copolymer (PEBAX® from Arkema, Inc.) and non-electroconductive polymeric materials such as polyethylene phthalate (PET), polytetrafluoroethylene (PTFE), fluorinated ethylene propylene (FEP), perfluoroalkoxy (PFA), urethane, polyurethane, or polyvinyl chloride (PVC). The tube 51 may or may not be transparent.

The tube 51 has a certain flexibility (flexuosity), incompressibility along the tube axis, and torsional stiffness. The torsional stiffness of the tube 51 allows transfer of rotative torque from the control handle (not shown) to the head portion 40.

The optical fiber cable 54, the optical fiber cable (not shown) continuous with the light diffuser for monitoring 45, and the ten lead wires 48 extend to a connector (not shown) via the control handle (not shown) connected to a proximal portion of the tubular portion 50. The connector (not shown) connects the optical fiber cable 54, the optical fiber cable (not shown) continuous with the light diffuser for monitoring 45, and the ten lead wires 48 to an end of a tube (not shown) connected to the treatment apparatus 1.

«Use of Laser Catheter 30 for Treatment and Measurement of Treatment Depth»

Now, a process for using the laser catheter 30 of this embodiment to determine information on a photodynamic therapy for arrhythmias and the extent of lesions in the photodynamic therapy by the treatment apparatus 1 will be described.

First, the laser catheter 30 is inserted through the femoral vein or the cervical vein into the heart of a patient (not shown) by a clinician such as a doctor. The distal end portion of the laser catheter 30 is brought into contact with the inner peripheral side wall of myocardial tissue of the left atrium around the pulmonary vein.

Then, a required therapeutic amount of a PDT agent is intravenously administered to the patient at one time by the clinician. The administered PDT agent diffuses into interstitial spaces or blood vessels.

After a predetermined period in the range of, for example, from several minutes to several tens of minutes of administration of the PDT agent, a switch (not shown) of the light source 11 is turned on by the clinician to cause the light source 11 to start radiation of excitation light, which is then transmitted through the entire length of the light diffuser for irradiation 44 of the laser catheter 30.

When the switch of the light source 11 is turned on at the time of start of light irradiation, a timer (not shown) is turned on.

The control unit 14 calculates the intensity of fluorescence detected by the fluorescence detection unit 12 when the light source 11 is turned on and stores the intensity in a memory (not shown).

Then, the control unit 14 determines whether $\Delta t$ (sec) (in the range of, for example, from 0.05 seconds to 0.5 seconds), which is a predetermined measurement time interval, has elapsed after the intensity of fluorescence is calculated. When Δt (sec) has elapsed, the control unit 14 calculates the intensity of fluorescence detected by the fluorescence detection unit 12 at that point in time and stores the intensity in a memory (not shown).

The control unit 14 acquires a light irradiation time, which indicates a period from the time of start of light irradiation to that point in time, from the timer (not shown). The control unit 14 calculates the intensity of returned excitation light detected by the returned excitation light detection unit 13 at that point in time and calculates estimated treatment-depth $d_{nec}$ using the calculated fluorescence intensity at that point in time and the above-mentioned Formulas 1 and 2.

Then, the control unit 14 transmits the data of the calculated estimated treatment-depth $d_{ncc}$ to the display unit 16 and stores the data in a memory (not shown).

This allows the display unit 16 to present the estimated treatment-depth $d_{ncc}$ in real time so that the data is visible to the clinician.

In this case, the estimated treatment-depth $d_{ncc}$ is not the absolute depth of the tissue lesions created by the photodynamic therapy, but an index determined from relative values. For example, the estimated treatment-depth $d_{ncc}$ is an integer or a number with 1 or 2 decimal places such as "3" or "5.3".

In place of the estimated treatment-depth $d_{ncc}$, a corrected PBI corrected by a method for correction of PBI using histophysiological information obtained by another process may be used to calculate a corrected estimated treatment-depth $d_{ncc}$, which may be then displayed and stored.

Although PBI or a corrected PBI is used as an index of information on the extent of lesions by photodynamic therapy in this embodiment, the index is not limited thereto, and the intensity of fluorescence detected by the fluorescence detection unit 12 may be used as information on the extent of lesions. In this case, the intensity of fluorescence with or without modification is displayed on the display unit 16 and stored in a memory (not shown).

Then, the control unit 14 determines whether a predetermined measurement time has elapsed or whether the light source 11 has been turned off. If the measurement time has not elapsed, and the light source 11 has not been turned off, computation and presentation of the estimated treatment depth $d_{ncc}$ are repeated.

If the predetermined measurement time has elapsed, or the light source 11 has been turned off, the control unit 14 determined that the photodynamic therapy is complete. Then the control unit 14 stores the data stored in a memory (not shown) in the memory unit 17 and terminates the processing.

REFERENCE SIGNS LIST

| | |
|---|---|
| A: | central axis |
| C: | center |
| F: | force |
| L1, L2, r: | straight line |
| 1: | treatment apparatus |
| 11: | light source |
| 12: | fluorescence detection unit |
| 13: | returned excitation light detection unit |
| 14: | control unit |
| 15: | operation unit |
| 16: | display unit |
| 17: | memory unit |
| 20: | optical system |
| 21: | polarizing beam splitter |
| 22, 44f, 45f: | optical fiber |
| 23, 63: | dichroic mirror |
| 24: | band-pass filter |
| 30, 30': | laser catheter |
| 40, 40': | head portion |
| 40b: | bent portion |
| 40c: | loop portion |
| 40h: | side hole |
| 40o: | center |
| 40t: | distal end portion |
| 41: | transparent tube |
| 41i, 41o: | wall |

| 41w: | partition wall |
|---|---|
| 42: | inner peripheral side lumen |
| 43: | outer peripheral side lumen |
| 44: | light diffuser for irradiation |
| 44a: | light diffuser body |
| 44b: | resin layer |
| 44t: | distal end portion |
| 45: | light diffuser for monitoring |
| 46: | reflector |
| 46a', 46b': | reflector coil |
| 47: | shape-memory wire |
| 48: | lead wire |
| 49h: | fine hole |
| 49C, 49C1: | coil electrode |
| 49P: | plating film |
| 49R: | ring electrode |
| 49T: | end electrode |
| 50: | tubular portion |
| 51: | tube |
| 54: | optical fiber cable |
| 54c: | cladding |
| 54d: | coating |

**Claims**

1. A medical device for treating a site to be treated by inserting the medical device into a living body and irradiating the site with light,
   wherein the medical device comprises a distal end portion that has a long tube and a long outer wall of the distal end portion, the wall being provided with electrodes exposed on the outer surface of the tube, and long light-diffusers disposed inside the outer wall of the distal end portion, and
   wherein the distal end portion comprises a light transmission equalizer that equalizes light transmission along a length direction of the outer wall of the distal end portion.

2. The medical device according to claim 1, wherein the electrodes comprise a plurality of approximately annular elements that are spaced along the length direction of the distal end portion over the entire distal end portion extending from one end in the length direction to the other.

3. The medical device according to claim 1 or 2, wherein the electrodes have a length of 1 mm or less along the length direction of the distal end portion.

4. The medical device according to claim 2 or 3, wherein the approximately annular elements are annular elements that are provided with a plurality of voids penetrating through the annular elements.

5. The medical device according to claim 4, wherein the voids are holes or slits.

6. The medical device according to claim 4 or 5,
   wherein a blocking member that blocks light diffused by the light diffuser is disposed in the tube along the length direction, and
   wherein the voids are disposed only on the side extending from the blocking member to the light diffuser.

7. The medical device according to any one of claims 4 to 6,
   wherein the distal end portion comprises a loop portion that has a coil shape having one and a few convolutions when the loop portion is subject to no external stresses and a bent portion that is formed at an end of the loop portion, and
   wherein the voids are formed only on the outer peripheral side of the coil shape.

8. The medical device according to claim 2, wherein the approximately annular elements are formed of a coil having a wire diameter of from 0.05 to 1 mm.

9. The medical device according to claim 8, wherein the coil has one or more convolutions or has a pitch that is larger than the wire diameter.

10. The medical device according to claim 8 or 9,
   wherein the tube comprises spiral projections formed along the outer circumference of the tube,
   wherein the projections are spaced in a plural number on the outer surface of the tube, along the length direction of the distal end portion over the entire distal end portion extending from one end in the length direction to the other, and
   wherein the electrodes are fixed to the projections.

11. The medical device according to claim 1 or 2, wherein the electrodes are light-transmissive metal films that are continuously disposed along the direction of extension of the tube and has a film thickness of from 1 to 300 nm.

12. A phototherapy system comprising:

   the medical device according to any one of claims 1 to 11,
   a light source that provides excitation light to the light diffuser for irradiation of the medical device,
   a detection unit that detects the electric potential of the electrodes, and
   a calculator that receives a signal indicative of the detected electric potential from the detection unit and calculates the electric potential of the electrodes based on the signal.

FIG. 1

LASER CATHETER

49T,49R          45          44          30

| ELECTRODES | LIGHT DIFFUSER FOR MONITORING | LIGHT DIFFUSER FOR IRRADIATION |

ELECTRIC POTENTIAL DETECTION UNIT — 18

15 — OPERATION UNIT

CONTROL UNIT — 14

16 — DISPLAY UNIT

EXCITATION LIGHT DETECTION UNIT — 13

1

17 — MEMORY UNIT

FLUORESCENCE DETECTION UNIT — 12

20

21

24  23  22

LIGHT SOURCE — 11

FIG. 2

# FIG. 3

FLUORESCENCE INTENSITY
AT LIGHT IRRADIATION START TIME

FLUORESCENCE DETECTOR OUTPUT [V]

FIRST LIGHT
EMISSION
PERIOD

SECOND LIGHT
EMISSION PERIOD
(STEADY STATE)

RATIO BETWEEN FLUORESCENCE INTENSITY
AT LIGHT IRRADIATION START TIME
AND FLUORESCENCE INTENSITY AT STEADY STATE

IRRADIATION
TIME [S]

TIME OF FIRST LIGHT EMISSION PERIOD

# FIG. 4

FIG. 5

# FIG. 6

PROXIMAL SIDE

40

L1

α

L2

43

46

42

48

45

47

OUTER
PERIPHERAL
SIDE

INNER
PERIPHERAL
SIDE (C)

44

40o

41o    41w    41i

41

DISTAL SIDE

# FIG. 7

# FIG. 8

# FIG. 9

# FIG. 10

# FIG. 11

# FIG. 12

# FIG. 13

# FIG. 14

FIG. 15

FIG. 16

# FIG. 17

FIG. 18

# FIG. 19

## INTERNATIONAL SEARCH REPORT

| International application No. |
| --- |
| PCT/JP2014/080516 |

| A. CLASSIFICATION OF SUBJECT MATTER |
| --- |
| *A61B18/20*(2006.01)i |

According to International Patent Classification (IPC) or to both national classification and IPC

| B. FIELDS SEARCHED |
| --- |

Minimum documentation searched (classification system followed by classification symbols)
A61B18/20

Documentation searched other than minimum documentation to the extent that such documents are included in the fields searched

| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

| C. DOCUMENTS CONSIDERED TO BE RELEVANT |
| --- |

| Category* | Citation of document, with indication, where appropriate, of the relevant passages | Relevant to claim No. |
| --- | --- | --- |
| Y | JP 2010-536475 A (Cardiac Pacemakers, Inc.), 02 December 2010 (02.12.2010), paragraphs [0085] to [0100]; fig. 1, 6 & US 2009/0054883 A1 & WO 2009/025826 A1 | 1-12 |
| Y | JP 2012-506569 A (Advanced Photodynamic Technologies, Inc.), 15 March 2012 (15.03.2012), entire text; all drawings & US 2010/0097822 A1 & WO 2010/047750 A1 & CA 2740373 A & CN 102203646 A | 1-12 |

☒ Further documents are listed in the continuation of Box C.  ☐ See patent family annex.

| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 05 December, 2014 (05.12.14) | 16 December, 2014 (16.12.14) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

36

**INTERNATIONAL SEARCH REPORT**

International application No.

PCT/JP2014/080516

C (Continuation). DOCUMENTS CONSIDERED TO BE RELEVANT

| Category* | Citation of document, with indication, where appropriate, of the relevant passages | Relevant to claim No. |
|---|---|---|
| Y | JP 11-511999 A (Board of Regents, the University of Texas System), 19 October 1999 (19.10.1999), page 13; fig. 2 & US 5824005 A & US 6143019 A & US 6736808 B1 & WO 1997/007735 A1 & CN 1193267 A | 2-12 |
| Y | JP 2010-155083 A (Biosense Webster, Inc.), 15 July 2010 (15.07.2010), paragraphs [0035], [0036]; fig. 5, 8 & JP 2011-224373 A & US 2010/0168548 A1 & US 2010/0222859 A1 & EP 2229904 A1 & EP 2380518 A2 & CA 2688973 A & CN 101766502 A & CA 2737678 A & CN 102274021 A & CA 2688973 A1 | 4-7,10,12 |
| Y | JP 2008-501441 A (Edwards Lifesciences Corp.), 24 January 2008 (24.01.2008), paragraphs [0038] to [0052]; fig. 1, 2 & JP 2008-501444 A & US 2005/0273090 A1 & US 2005/0288654 A1 & WO 2005/120379 A2 & WO 2006/007305 A2 & CA 2569214 A & CA 2569582 A | 7,12 |
| A | JP 10-504989 A (Rare Earth Medical, Inc.), 19 May 1998 (19.05.1998), entire text; all drawings & US 5643253 A & US 5637877 A & WO 1996/007451 A2 | 1-12 |

Form PCT/ISA/210 (continuation of second sheet) (July 2009)

**EP 3 072 470 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2012034852 A
- JP 2008501441 A
- JP 2961074 B